Europäisches Patentamt

European Patent Office

Office européen des brevets

(19)

(11) Publication number: **0 118 928**
**B1**

# EUROPEAN PATENT SPECIFICATION

(12)

(45) Date of publication of the patent specification:
29.10.86

(51) Int. Cl.⁴: **C 12 P 1/00**

(21) Application number: **84200011.9**

(22) Date of filing: **06.01.84**

(54) Method for the enzymatic conversion of a water-insoluble or substantially water-insoluble organic substrate.

(30) Priority: **03.02.83 NL 8300393**

(43) Date of publication of application:
**19.09.84 Bulletin 84/38**

(45) Publication of the grant of the patent:
**29.10.86 Bulletin 86/44**

(84) Designated Contracting States:
**AT BE CH DE FR GB IT LI LU NL SE**

(73) Proprietor: **DUPHAR INTERNATIONAL RESEARCH B.V,
C.J. van Houtenlaan 36, NL-1381 CP Weesp (NL)**

(72) Inventor: **Laane, Nicolaas C. M., c/o OCTROOIBUREAU
ZOAN B.V. Apollolaan 151, NL-1077 AR Amsterdam (NL)**
Inventor: **Hilhorst, Maria H., c/o OCTROOIBUREAU
ZOAN B.V. Apollolaan 151, NL-1077 AR Amsterdam (NL)**
Inventor: **Veeger, Cornelis, c/o OCTROOIBUREAU
ZOAN B.V. Apollolaan 151, NL-1077 AR Amsterdam (NL)**
Inventor: **Grande, Henk J., c/o OCTROOIBUREAU ZOAN
B.V. Apollolaan 151, NL-1077 AR Amsterdam (NL)**

(74) Representative: **Swaters, Pieter D. et al,
OCTROOIBUREAU ZOAN B.V. P.O. Box 140, NL-1380 AC
Weesp (NL)**

(56) References cited:
EP - A - 0 068 594
WO - A - 82/01563
US - A - 3 926 726

LETTERS-FEDERATION OF EUROPEAN BIOCHEMICAL
SOCIETIES, vol. 159, nos. 1,2, August 1983, pages
225-228, Elsevier Science Publishers B.V., Amsterdam,
NL R. HILHORST et al.: "Enzymatic conversion of
apolar compounds in organic media using an
NADH-regenerating system and dihydrogen as
reductant"
RUSSIAN CHEMICAL REVIEWS, translated from
Uspekhi Khimii, vol. 49, no. 5, May 1980, pages 385-403,
London, GB K. MARTINEK et al.: "The stabilisation of
enzymes - a key factor in the practical application of
biocatalysis"
JOURNAL OF SOLID-PHASE BIOCHEMISTRY, vol. 5, no.
4, 1980, pages 269-282, Plenum Publishing Corp., New
York, US P. MEIER et al.: "Micellar solubilazation of
biopolymers in hydrocarbon solvents. II. The case of
horse liver alcohol dehydrogenase"

(56) References cited: (continuation)
BULLETIN DE LA SOCIETE CHIMIQUE DE FRANCE, part
II, nos. 1-2, 1980, pages 98-100, Paris, FR M.D. LEGOY et
al.: "Utilisation des hydroxystéroides déshydrogénases
en milieu non aqueux. Recyclage du cofacteur
pyridinique"
ZEITSCHRIFT FÜR ALLGEMEINE MIKROBIOLOGIE, vol.
22, no. 9, 1982, pages 607-615 F. ERGAN et al.: "Steroid
modifications with immobilized biocatalysts - use of
immobilized enzyme-requiring cofactor regeneration
and of immobilized mycelium"
PROCEEDINGS OF THE NATIONAL ACADEMY OF
SCIENCES OF THE UNITED STATES OF AMERICA, vol.
79, no. 12, June 1982, pages 3927-3930, Washington, US
R. HILHORST: "Photosensitized production of
hydrogen by hydrogenase in reversed micelles"
EUR. J. BIOCHEM 144 459-466 (1984)

The file contains technical information submitted after
the application was filed and not included in this
specification

## Description

The invention relates to a method of converting a water-insoluble or substantially water-insoluble organic substrate under the influence of a water-soluble enzyme requiring a cofactor, by performing the conversion in a micellar system, comprising an organic solvent which is not or substantially not water-miscible and in which the enzyme is solubilized via reversed micelles, which are stabilized by one or more surfactants. Water-insoluble or substantially water-insoluble is to be understood to mean herein a solubility of at most approximately 3 g per litre of water at room temperature. An organic substrate is to be understood to mean herein an organic compound which can be converted specifically or aspecifically by the enzyme.

Such a conversion is known from literature. For example Meier and Luisi (J. Solid-Phase Biochem., Vol. 5, No. 4, 1980, 269–282) have reduced decanal enzymatically in a system having a hydrocarbon as a solvent; a steroid containing a ketone function can be reduced in the same manner. In this non-water-miscible organic solvent tiny waterpools, stabilized by surfactants, are present, so-called reversed micelles. The enzyme is present in the micellar phase. Solubilized via reversed micelles is hence to be understood to mean herein that the enzyme is present in the organic solvent in the micellar phase (i.e. waterpool plus interphase). The total system of enzyme, micellar phase and organic solvent is termed micellar system. Most enzymes are not soluble in organic solvents and are moreover easily denaturated by the solvent. Therefore, such a system of reversed micelles is often necessary to enable an enzymatic process in an organic solvent.

Meier and Luisi have described an enzymatic reduction of decanal which is catalysed by the enzyme liver alcohol dehydrogenase with NADH as a cofactor. The enzyme described by said authors, like many other enzymes, requires a cofactor for its enzymatic activity. Cofactors, also termed coenzymes, for example NADH and NADPH for reduction reactions and $NAD^+$ and $NADP^+$ for oxidation reactions, however, are very expensive organic compounds and, in solution, are often unstable (see, for example, Wong and Whitesides, J. Am. Chem. Soc. 103, 1981, 4890–99). If such an enzymatic process should be economically feasible it is hence necessary to regenerate the cofactor.

The regeneration of cofactors is known, for example, from a survey article by Wang and King in Adv. Biochem. Eng. 12. 1979, 119–146. More recently, several articles have been published, for example, by Wong c.s., which all relate to the regeneration of the NAD(P)H cofactor: J. Org. Chem. 46, 1981, 4622–23, J. Am. Chem. Soc. 103, 1981, 4890–99 and 6227–28. Enzymatic methods are preferred over chemical methods in regenerating cofactors, in particular because the regeneration occurs with a comparatively small efficiency when a chemical method is used. Such an enzymatic regeneration of a cofactor is known only in aqueous systems.

On the basis of the above it would be possible in principle after the enzymatic conversion of the organic substrate to isolate the cofactor to be regenerated from the micellar system and subsequently to regenerate the cofactor in a known manner in an aqueous medium. The regenerated cofactor would then be available again for the desired conversion.

It will be obvious that such a separate regeneration has important drawbacks. A separate reaction step is expensive, requires a much higher cofactor concentration and is time-consuming. Moreover the isolation of the cofactor to be regenerated from the micellar system is usually extremely difficult.

It is the purpose of the invention to perform the enzymatic process mentioned in the opening paragraph in an economically acceptable manner.

According to the invention this object is achieved in that the cofactor is regenerated in situ by means of one or more second enzymes, which are also solubilized via reversed micelles in the organic solvent.

In this way the laborious isolation of the cofactor to be regenerated succeeded by a separate regeneration process can be avoided.

In US patent 3 926 726 of Antonini et al. a method for carrying out enzymatic reactions by using a biphasic aqueous-organic system is described; the cofactor can be regenerated in situ. This known biphasic system differs fundamentally from the micellar system of the invention, in which system the enzymes are solubilized via reversed micelles. So the system of the present invention should be considered as a solution and not as a biphasic system. Although in some experiments apparently high conversions could be obtained, the method described in the above US patent is generally less broadly applicable. This is obvious from e.g. a publication of the same Antonini and co-workers in Biotechn. Bioeng. Vol XVII, pp. 1101–1108 (1975), wherein is stated, that in reducing progesterone poor results were obtained in spite of the relative high specificity of 20β-HSDH for this substrate. As will be clear from the Examples, progesterone could be reduced in a high yield and specificity to 20β-hydroxypregn-4-ene-3- one, when the method of the invention was used. Therefore the method of the present invention has considerable advantages over the above-mentioned known reaction in a biphasic system.

To gain a better insight into the method of the present invention, the micellar system used in the invention is described in more detail hereafter. The reversed micelles which preferably consist of tiny waterpools, approx. 2 to 200 nm in diameter and stabilised by one or more surfactants, are present in the organic solvent. Ionic surfactants are to be preferred, for example, ammonium salts or alkali metal salts of carboxylic acids, or quaternary ammonium salts; the carboxylic acid and the quaternary ammonium salt must comprise at least one aliphatic chain having at least 8 carbon atoms. Examples of suitable ionic surfactants are dodecyl ammonium propionate (DAP), cetyl trimethyl ammonium bromide (CTAB) and dioctyl sodium sulphosuccinate (AOT).

It has furthermore been proved to be very advantageous, when the micellar system in addition comprises a so-called cosurfactant, wherein the substrate is well soluble. A cosurfactant is to be understood to mean herein a substance which can influence the micellar system in such a manner that the desired enzymatic conversion is promoted. A substance having a large dipole moment, for example, an aliphatic or cycloaliphatic alcohol having 3 to 10 carbon atoms, such as a butanol, pentanol, hexanol or cyclohexanol, is preferably used as a cosurfactant; other suitable cosurfactants are amines, fatty acids or derivatives thereof, or other non-ionized amphiphilic substances. Of course, the cosurfactant should be inert in the system in which the desired conversion is carried out, unless, as will be explained hereinafter, the cosurfactant is used as a substrate.

The choice of the cosurfactant depends on the substrate to be converted, in the sense that the cosurfactant should be a good solvent for the substrate. By way of example, for progesterone as a substrate hexanol is very suitable as a cosurfactant, whereas for prednisone butanol or pentanol is to be preferred. The cosurfactant concentration to be used depends considerably on the concentration and nature of the other ingredients of the micellar system. Generally, however, the cosurfactant is used in a quantity of 5–40% v/v, calculated with respect to the amount of organic solvent present in the system. The cosurfactant molecules serve to reduce the partition coefficient of the substrate, i.e. the ratio of the concentrations of the substrate, i.e. the ratio of the concentrations in the organic phase and in the micellar phase. As a consequence the rate of the enzymatic conversion is promoted. It has further been proved, that a relatively large number of cosurfactant molecules in the interphase of the reversed micelles also favours the desired conversion. Each reversed micelle is stabilised by surfactant molecules in the interface. The molecular ratio between cosurfactant and surfactant in the interphase of the reversed micelles should therefore generally be 0.1 to 20, preferably 1–6.

The choice of the organic solvent should preferably be adapted to the surfactant and the cosurfactant to be used. Suitable solvents for the conversion according to the invention are halogenated or non-halogenated hydrocarbons or mixtures thereof, preferably benzene substituted optionally with one or more chlorine atoms or alkyl groups, for example, chlorobenzene, toluene or xylenes, a chlorinated aliphatic hydrocarbon having 1–14 carbon atoms, for example, chloroform, carbontetrachloride or trichloroethane, a dialkyl ether the alkyl groups of which each have 4–6 carbon atoms, a cyclic or non-cyclic alkane having at least 4, preferably 6–12 carbon atoms, for example straight or branched heptane or octane, or a mixture of two or more of these solvents.

The temperature at which the enzymatic conversion according to the invention is carried out has an upper limit imposed by the temperature sensitivity of the enzymes used and a lower limit imposed by the stability of the micellar system. The process is preferably carried out at room temperature or a slightly elevated temperature. The ratio of the quantity of water and the other ingredients of the micellar system is rather critical and depends on the nature and the properties of these ingredients. In general it holds that optimum results can be achieved with approximately that quantity of water, that gives maximal hydration of all the hydrophilic head-groups of the surfactant or surfactants.

In evaluating the invention it should be considered that a system of reversed micelles is an extremely subtle reaction medium in which small changes in the composition may have a dramatic influence on the stability of the system, and may hence empede or prevent the conversion of the substrate. In fact, prime requirements for a successfully occurring conversion are both the stability of the micellar system and the accessibility of the substrate in the organic solvent for the enzymes in the reversed micelles. It is therefore an achievement of the invention that one has succeeded to satisfactorily perform the enzymatic regeneration of the cofactor in this subtle micellar system without adversely influencing the desired enzymatic conversion, namely due to a correct choice of the nature and the composition of the ingredients of the micellar system.

Various conversion reactions, for example, oxidations, reductions and hydroxylation reactions may be carried out by means of the method according to the invention. It is a characteristic of these enzymatic conversions, that the reactions can be performed with a high site- and stereospecificity.

When the method is used to oxidise a water-insoluble or substantially water-insoluble organic substrate, $NAD^+$ or $NADP^+$ is preferably used as a cofactor which is also solubilized via reversed micelles in the organic solvent. In such an oxidation reaction, an electron acceptor is usually present in the micellar system. If the reaction is not carried out while excluding air, the air oxygen may serve as an electron acceptor. However, it may also be desirable to use an artificial electron acceptor, preferably a viologen, a phenazine derivative or an acridine derivative, such as methyl viologen, benzyl viologen, methylene blue or phenazine methosulphate, usually in the presence of molecular oxygen, or to introduce oxygen into the system. If the oxidation reaction is carried out in the absence of oxygen, the artificial electron acceptor may be oxidised electrochemically, so by using an oxidative voltage source. Suitable second enzymes for such an oxidation reaction are catalase in the presence of superoxide dismutase, NAD(P)H-oxidase and NAD(P)H-dehydrogenase. These enzymes can efficaciously catalyse the regeneration of the above cofactors.

When the method according to the invention is used for reducing a water-insoluble or substantially water-insoluble organic substrate, NADH or NADPH which, like the enzymes, is solubilised via reversed micelles in the organic solvent, is preferably used as a cofactor. In such a reduction reaction an electron donor should preferably be present in the system. An electron donor suitable for this purpose is a reduced viologen, preferably reduced methyl viologen ($MV^+$) or benzyl viologen. For regenerating the cofactors NADH or NADPH used in the reduction lipoamide dehydrogenase or ferredoxine-NADP$^+$ oxidoreductase as a second enzyme have proved to be excellently suitable.

Reduced methyl viologen ($MV^+$) or benzyl viologen is preferably obtained by subjecting methyl viologen ($MV^{2+}$) or benzyl viologen in situ, hence in the system itself, to an electrochemical reduction, a photochemical reduction or a reduction with hydrogen gas under the influence of the enzyme hydrogenase. Methyl or benzyl viologen then is reduced efficiently. The active enzyme hydrogenase which is isolated from Desulfovibrio vulgaris according to the method described in Netherlands Patent Application published as No 7801517 has proved suitable for the enzymatic reduction of methyl or benzyl viologen.

Enzymes suitable for the above-mentioned oxidation and reduction reactions and requiring a cofactor are alcohol dehydrogenases and hydroxysteroid dehydrogenases, for example, liver alcohol dehydrogenase, α-hydroxysteroid dehydrogenase, β-hydroxysteroid dehydrogenase, and 3α-20β-hydroxysteroid dehydrogenase.

The oxidation and reduction reactions described above are particularly suitable for the oxidation of a substrate comprising one or more aldehyde-, ketone- or alcohol groups, or for the reduction of a substrate comprising one or more aldehyde- or ketone groups. Examples of suitable substrates of the oxidation reaction are testosterone, androsterone, pregnenolone and (substantially) water-insoluble aliphatic aldehydes and alcohols, for the reduction reaction: progesterone, cortisone, prednisone, pregnenolone, and (substantially) water-insoluble aliphatic aldehydes and ketones.

As a particular aspect of the invention it was found that in the enzymatic oxidation of an aliphatic alcohol the substrate may also serve as a cosurfactant, hence may form an integral part of the micellar system. In that case the alcohol concentration in the organic phase should be between 5 and 50% v/v. Substrates suitable for this purpose are aliphatic alcohols having 3 to 10 carbon atoms, for example, a butanol, a pentanol or a hexanol. It has proved possible in such a system to oxidise pentanol and hexanol in octane or toluene as an organic solvent and with CTAB as a surfactant to the desired aldehydes; in these experiments the alcohol and the hydrocarbon solvent were present in a volume ration of 24:100.

After the desired conversion, the resulting product is isolated from the reaction mixture in a manner known per se. Usually, the enzymes are first recovered, for example, by extracting the micellar system with water or an aqueous solution. Usually, the surfactant or surfactants is or are then removed from the micellar system, preferably by precipitation with a suitable organic solvent, succeeded by filtration or centrifugation. The resulting product is finally isolated from the system, for example, by filtration over one or more micropore filters or by extraction with an organic solvent or mixture of solvents suitable for this purpose, for example dichloromethane, acetonitrile, or a mixture of one of these solvents with another organic solvent. The precipitation of the surfactant or surfactants is preferably carried out simultaneously with the extraction of the product by means of a carefully chosen solvent or mixture of solvents. The above-mentioned organic solvents or mixtures of solvents are well suitable for this double function. The resulting final product may, if so desired, be isolated in the pure state by means of methods known for this purpose, for example, distillation or recrystallization, or by means of column chromatography.

The progress of the conversion may be followed by methods likewise known for this purpose, for example, by means of a chromatographic method, for example, gas-liquid chromatography or thin-layer chromatography, or by means of IR- or UV-spectrophotometry.

The invention will be described in greater detail with reference to the following specific examples.

Example I

A steroid, namely progesterone (pregn-4-ene-3,20-dione) was reduced as follows.

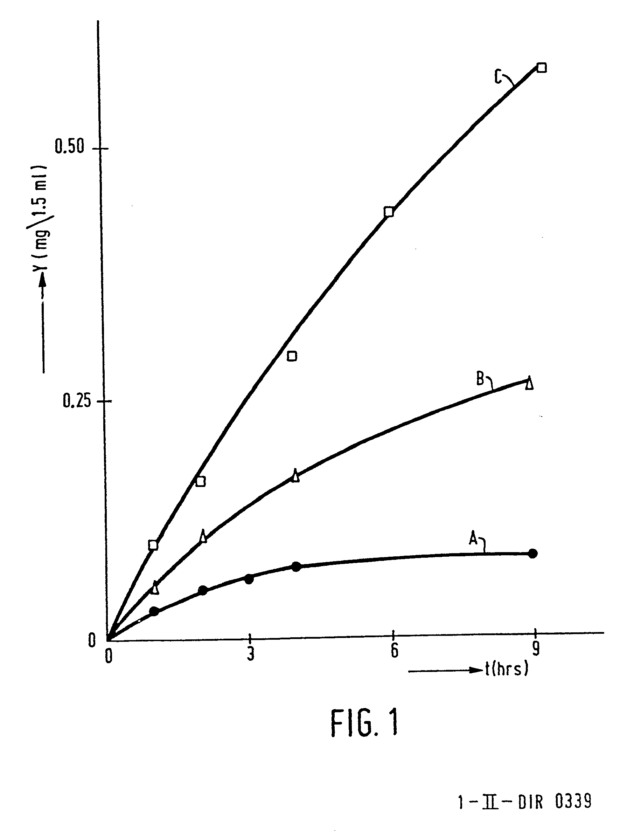

1.5 ml of an 0.2 M solution of cetyl trimethyl ammonium bromide (CTAB) in a mixture of octane and hexanol in a volume ratio of 4:1, in which in addition 1 mM of progesterone had been dissolved, was mixed, while stirring intensively, with 60 µl of a buffer solution of 50 mM of N-2-hydroxyethylpiperazine N'-2-ethane sulphonic acid (HEPES) in dilute sodium hydroxide solution (HEPES-buffer) with a pH of 7.6. A 1 M solution is to be understood to mean herein a 1 molar solution which is a solution of 1 grammolecule of substance in 1 litre of solvent. The buffer solution comprised 1.0 mM of NAD$^+$, 25 mM of methyl viologen, 5 µg of lipoamide dehydrogenase (EC 1.6.4.3) and 19 µg of 20β-hydroxysteroid dehydrogenase (EC 1.1.1.53). The micellar system thus obtained was made oxygen-free with argon, after which the argon was replaced by hydrogen. In all the subsequent treatments the system was then kept under hydrogen. Then 10 µl of a solution of 29 µg/ml of hydrogenase, isolated from Desulfovibrio vulgaris as described in the already mentioned Netherlands Patent Application published as No 7801517, in HEPES-buffer were added to the system while stirring, after which the reaction system was incubated at 25 °C. At intervals a sample was taken from the reaction system and, after removing CTAB by precipitation with acetonitrile, analysed by means of HPLC (high performance liquid chromatography). The results of these analyses are shown graphically in Figure 1. In graph B the yield of 20β-hydroxypregn-4-ene-3-one (Y) in mg per 1.5 ml is plotted against the time in hours. Only the β-isomer, and not the corresponding α-isomer, viz. 20α-hydroxypregn-4-ene-3-one, was found in the reaction mixture. So progesterone was reduced site- and stereospecifically. The ratio hexanol/CTAB in the interphase of the reversed micelles was under these conditions 2.5.

When instead of 1.0 mM of progesterone 0.2 mM or 5.0 mM of progesterone was used as a starting material, under the same conditions the results presented in curve A or curve C were obtained.

The in situ formation of reduced methyl viologen may also take place photochemically or electrochemically instead of enzymatically. The photochemical reduction was carried out as described in an article by Hilhorst et al. in Proc. Natl. Acad. Sci. USA, 79 (1982), 3927–3930. The electrochemical reduction was carried out as described in Example III.

Both the electrochemical and the photochemical reduction yielded the same results as the enzymatic reduction described above.

Example II

Influence of cosurfactant on conversion of progesterone.

The reduction of progesterone according to Example I was repeated with various cosurfactants. Under the same reaction conditions as described in Example I 1 mM of progesterone was reduced to 20β-hydroxypregn-4-ene-3-one in the presence of 180 µl of propanol, 220 µl of butanol, 250 µl of pentanol, 300 µl of hexanol and 375 µl of octanol, respectively as cosurfactants. In each of these experiments the organic phase was supplied with octane up to a total volume of 1.5 ml.

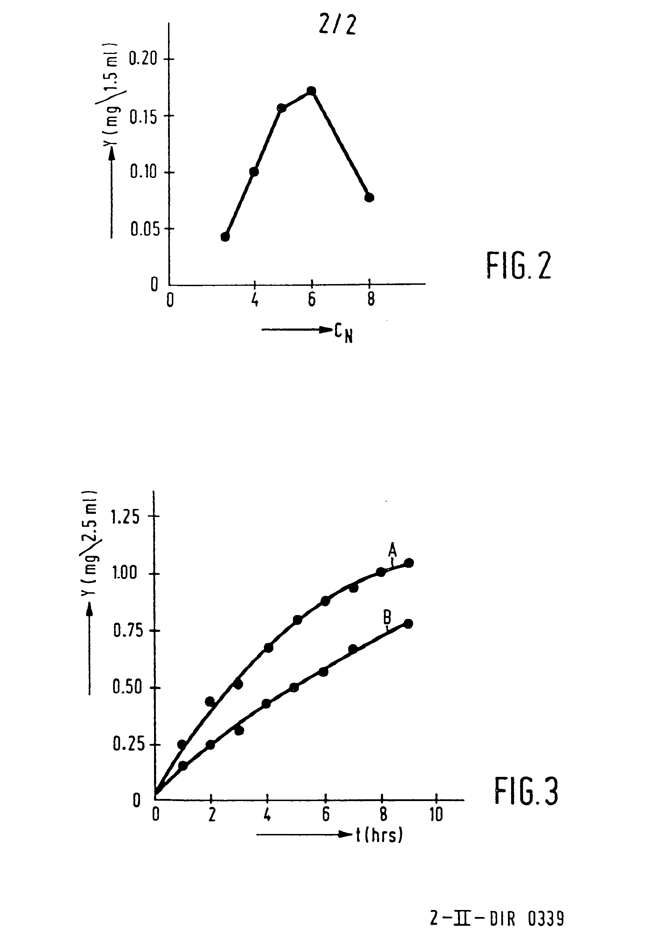

The results are shown graphically in Figure 2. In this graph the yield (Y) of 20β-hydroxypregn-4-ene-3-one in mg per 1.5 ml, formed after a reaction time of 4 hours, is plotted against the chain length ($C_n$) of the alcohol used as a cosurfactant. From this graph it is clear, that hexanol and pentanol are most suitable as cosurfactants for the desired conversion.

Example III

Oxidation of testosterone to androstenedione; electrochemical in situ formation of benzyl viologen.

The following ingredients were mixed thoroughly: 0.06 g of tetraheptyl ammonium bromide, 0.18 g of cetyl trimethyl ammonium bromide (CTAB), 250 µl of hexanol, 2 ml of octane, 10 µl of a solution of 50 mM benzyl viologen in 200 mM HEPES-buffer (pH 7.6), and 20 µl of 25 mM $NAD^+$ in 200 mM HEPES-buffer (pH 7.6). To the micellar system thus obtained was added 5 µmoles of testosterone. This mixture was introduced into the anodic part of an electrochemical cell. Into the same cell but separated from this mixture by a dialysis membrane another mixture was introduced. This latter mixture comprised the same ingredients as the former, however without $NAD^+$ and testosterone. A platina electrode was used; the reference electrode was a Ag/AgBr electrode. The system was made oxygen-free with argon, after which the system was kept under argon. After the potential of the anode has been adjusted to +800 mV, 10 µl of lipoamide dehydrogenase (2 mg/ml) and 30 µl of 3α,β-hydroxysteroid dehydrogenase (30 mg/ml) (EC 1.1.1.52) were added. The addition of 30 µl of 3α,β-hydroxysteroid dehydrogenase was repeated after 3 and 6 hours. The reaction mixture was analyzed as described in Example I.

The results are shown graphically in Figure 3. In graph A the yield of androstenedione (Y) in mg per 2.5 ml is plotted against the time in hours.

The ratio hexanol/CTAB in the interphase of the reversed micelles was 2.5.

When instead of 250 µl of hexanol and 2 ml of octane 500 µl of hexanol and 1.75 ml of octane was used curve B in Figure 3 was obtained.

The ratio hexanol/CTAB in the interphase of the reversed micelles was in this case greater than 2.5, but the partition coefficient of the substrate was increased relative to the value in graph A due to the presence of more hexanol in the organic phase.

**Claims**

1. A method of converting a water-insoluble or substantially water-insoluble organic substrate under the influence of a water-soluble enzyme requiring a cofactor, by performing the conversion in a micellar system, comprising an organic solvent which is not or substantially not water-miscible and in which the enzyme is solubilized via reversed micelles, which are stabilized by one or more surfactants, characterized in that the cofactor is regenerated in situ by means of one or more second enzymes, which are also solubilized via reversed micelles in the organic solvent, and that the surfactant and the organic solvent are selected in such a way, that the partition coefficient of the substrate in the micellar system, i.e. the ratio of the concentrations of the substrate in the organic phase and in the micellar phase, is as small as possible.

2. A method of converting a water-insoluble or substantially water-insoluble organic substrate under the influence of a water-soluble enzyme requiring a cofactor, by performing the conversion in a micellar system, comprising an organic solvent which is not or substantially not water-miscible and in which the enzyme is solubilized via reversed micelles, which are stabilized by one or more surfactants, characterized in that the cofactor is regenerated in situ by means of one or more second enzymes, which are also solubilized via reversed micelles in the organic solvent, that the micellar system in addition comprises a cosurfactant, wherein the substrate is well soluble, and that the surfactant, the cosurfactant and the organic solvent are selected in such a way, that the partition coefficient of the substrate in the micellar system, i.e. the ratio of the concentrations of the substrate in the organic phase and in the micellar phase, is as small as possible.

3. A method as claimed in Claim 1 or 2, characterized in that an optionally halogenated hydrocarbon or a mixture of optionally halogenated hydrocarbons is used as an organic solvent and that the reversed micelles consist of tiny waterpools, approx. 2 to 200 nm in diameter and stabilised by one or more ionic surfactants.

4. A method as claimed in Claim 1 or 2, characterized in that as an organic solvent is used benzene optionally substituted with one or more chlorine atoms or alkyl groups, a chlorinated aliphatic hydrocarbon having 1–14 carbon atoms, an alkane or dialkyl ether having 6–12 carbon atoms, or a mixture of two or more of these solvents, and that the reversed micelles consist of tiny waterpools, approx. 2 to 200 nm in diameter and stabilised by one or more ionic surfactants.

**Patentansprüche**

1. Verfahren zum Umwandeln eines wasserunlöslichen oder im wesentlichen wasserunlöslichen organischen Substrats unter dem Einfluss eines wasserlöslichen Enzyms, das einen Cofaktor erfordert, durch Durchführung der Umwandlung in einem Mizellarsystem, umfassend ein organisches Lösungsmittel, das nicht oder im wesentlichen nicht mit Wasser mischbar ist und in dem das Enzym durch umgekehrte Mizellen, die durch ein oder mehrere Tenside stabilisiert sind, löslich gemacht wird, dadurch gekennzeichnet, dass der Cofaktor in situ mittels eines oder mehrerer zweiter Enzyme regeneriert wird, die ebenfalls über umgekehrte Mizellen im organischen Lösungsmittel löslich gemacht werden, und dass das Tensid und das organische Lösungsmittel derart ausgewählt werden, dass der Teilungskoeffizient des Substrats im Mizellarsystem, d.h. das Verhältnis der Konzentrationen des Substrats in der organischen Phase und in der Mizellarphase, so klein wie möglich ist.

2. Verfahren zum Umwandeln eines wasserunlöslichen oder im wesentlichen wasserunlöslichen organischen Substrats unter dem Einfluss eines wasserlöslichen Enzyms, das einen Cofaktor erfordert, durch Durchführen der Umwandlung in einem Mizellarsystem, das ein organisches Lösungsmittel umfasst, das nicht oder im wesentlichen nicht mit Wasser mischbar ist und in dem das Enzym über umgekehrte Mizellen, die durch ein oder mehrere Tenside stabilisiert sind, löslich gemacht wird, dadurch gekennzeichnet, dass der Cofaktor in situ mittels eines oder mehrerer zweiter Enzyme regeneriert wird, die ebenfalls über umgekehrte Mizellen im organischen Lösungsmittel löslich gemacht werden, dass das Mizellarsystem zusätzlich ein Cotensid umfasst, worin das Substrat gut löslich ist, und dass das Tensid, das Cotensid und das organische Lösungsmittel derart gewählt werden, dass der Teilungskoeffizient des Substrats im Mizellarsystem, d.h. das Verhältnis der Konzentrationen des Substrats in der organischen Phase und in der Mizellarphase, so klein wie möglich ist.

3. Verfahren, wie in Anspruch 1 oder 2 beansprucht, dadurch gekennzeichnet, dass ein gegebenenfalls halogenierter Kohlenwasserstoff oder eine Mischung von gegebenenfalls halogenierten Kohlenwasserstoffen als organisches Lösungsmittel verwendet wird und dass die umgekehrten Mizellen aus winzigen Wassertröpfchen, annähernd mit einem Durchmesser von 2 bis 200 nm und stabilisiert durch ein oder mehrere ionische Tenside, bestehen.

4. Verfahren wie in Anspruch 1 oder 2 beansprucht, dadurch gekennzeichnet, dass als organisches Lösungsmittel Benzol gegebenenfalls substituiert mit einem oder mehreren Chloratomen oder Alkylgruppen, ein chlorierter aliphatischer Kohlenwasserstoff mit 1 bis 14 C-Atomen, ein Alkan oder Dialkyläther mit 6 bis 12 C-Atomen oder eine Mischung von zwei oder mehreren dieser Lösungsmittel verwendet wird und dass die umgekehrten Mizellen aus winzigen Wassertröpfchen, annähernd mit einem Durchmesser von 2 bis 200 nm und stabilisiert durch ein oder mehrere ionische Tenside, bestehen.

**Revendications**

1. Procédé de conversion d'un substrat organique insoluble ou pratiquement insoluble dans l'eau sous l'action d'une enzyme soluble dans l'eau nécessitant un cofacteur, par réalisation de la conversion dans un systeme micellaire renfermant un solvant organique qui est non miscible ou pratiquement non miscible à l'eau et dans lequel l'enzyme est solubilisée par le biais de micelles inversées qui sont stabilisées par un ou plusieurs agents de surface, caractérisé en ce que le cofacteur est régénéré in situ à l'aide d'une ou plusieurs autres enzymes qui sont également solubilisées par l'intermédiaire de micelles inversées dans le solvant organique et que l'agent de surface et le solvant organique sont choisis de manière que le coefficient de partage du substrat dans le système micellaire, c.à.d. le rapport des concentrations du substrat dans la phase organique et dans la phase micellaire, soit aussi faible que possible.

2. Procédé de conversion d'un substrat organique insoluble ou pratiquement insoluble dans l'eau sous l'action d'une enzyme soluble dans l'eau nécessitant un cofacteur, par réalisation de la conversion dans un système micellaire renfermant un solvant organique qui est non miscible ou pratiquement non miscible à l'eau et dans lequel l'enzyme est solubilisée par le biais de micelles inversées qui sont stabilisées par un ou plusieurs agents de surface, caractérisé en ce que le cofacteur est régénéré in situ à l'aide d'une ou plusieurs autres enzymes qui sont également solubilisées par l'intermédiaire de micelles inversées dans le solvant organique, que le système micellaire comporte en outre un cosurfactif dans lequel le substrat est bien soluble et que l'agent de surface, le cosurfactif et le solvant organique sont choisis de manière que le coefficient de partage du substrat dans le système micellaire, c.à.d. le rapport des concentrations du substrat dans la phase organique et dans la phase micellaire, soit aussi faible que possible.

3. Procédé selon la revendication 1 ou 2, caractérisé en ce qu'un hydrocarbure éventuellement halogéné ou un mélange d'hydrocarbures éventuellement halogénés est employé comme solvant organique et que les micelles inversées sont constituées par de minuscules poches d'eau d'un diamètre d'environ 2 à 200 nm et stabilisées par un ou plusieurs agents de surface ionique.

4. Procédé selon la revendication 1 ou 2, caractérisé en ce qu'on emploi comme solvant organique du benzène éventuellement substitué par un ou plusieurs atomes de chlore ou groupes alkyles, un hydrocarbure aliphatique chloré possédant 1 à 14 atomes de carbone, un alcane ou un éther dialkylique possédant 6 à 12 atomes de carbone ou un mélange de deux ou davantage de ces solvants et que les micelles inversées sont constituées par de minuscules poches d'eau d'un diamètre d'environ 2 à 200 nm et stabilisées par un ou plusieurs agents de surface ioniques.

FIG. 1

0 118 928

2/2

FIG.2

FIG.3

2 — II — DIR 0339